# EUROPEAN PATENT SPECIFICATION

(11) **EP 1 434 603 B1**
(45) Date of publication and mention of the grant of the patent: **16.12.2009**
(21) Application number: 02789173.8
(22) Date of filing: 26.09.2002
(51) Int. Cl.: A61K 47/48, A61P 35/00

(54) **METHOD OF TREATMENT USING LIGAND-IMMUNOGEN CONJUGATES**
BEHANDLUNGSVERFAHREN MIT LIGANDEN-IMMUNOGENKONJUGATEN
METHODE DE TRAITEMENT UTILISANT DES CONJUGUES LIGAND-IMMUNOGENE

(30) Priority: 28.09.2001 US 325793 P; 01.10.2001 US 326322 P; 26.06.2002 US 391654 P
(43) Date of publication of application: 07.07.2004
(73) Proprietor: Purdue Research Foundation, West Lafayette, IN 47906 (US)
(72) Inventor: LOW, Philip, Stewart, West Lafayette, IN 47906 (US); LU, Yingjuan, West Lafayette, IN 47906 (US)
(74) Representative: Bannerman, David Gardner
(86) International application number: PCT/US2002/030546
(87) International publication number: WO 2003/028634

(56) References cited:
- EP-A1- 0 220 030
- WO-A-01/74382
- WO-A1-97/41831
- WO-A2-97/37690
- US-A- 5 417 982
- US-A- 5 547 668
- LU, YINGJUAN ET AL: "Folate targeting of haptens to cancer cell surfaces mediates immunotherapy of syngeneic murine tumors" CANCER IMMUNOLOGY IMMUNOTHERAPY , 51(3), 153-162 CODEN: CIIMDN; ISSN: 0340-7004, 2002, XP002347729
- LOW, PHILIP S. [REPRINT AUTHOR] ET AL: "Folate -mediated targeting of imaging and immunotherapeutic agents to tumors in vivo." PROCEEDINGS OF THE AMERICAN ASSOCIATION FOR CANCER RESEARCH ANNUAL MEETING, (MARCH, 2002) VOL. 43, PP. 947-948. PRINT. MEETING INFO.: 93RD ANNUAL MEETING OF THE AMERICAN ASSOCIATION FOR CANCER RESEARCH. SAN FRANCISCO, CALIFORNIA, USA. APRIL 06-10, 20, March 2002 (2002-03), XP002347730
- LOW, P. S. [REPRINT AUTHOR] ET AL: "Folate -mediated delivery of therapeutic and imaging agents to cancer tissue in vivo." BRITISH JOURNAL OF PHARMACOLOGY, (NOVEMBER, 2001) VOL. 134, NO. PROCEEDINGS SUPPLEMENT, PP. 178P. PRINT. MEETING INFO.: MEETING OF THE BRITISH PHARMACOLOGICAL SOCIETY. DUBLIN, IRELAND. JULY 03-05, 2001. BRITISH PHARMACOLOGICAL SOCIETY. CODEN: BJPCBM., November 2001 (2001-11), XP008053391
- ROY E.J. ET AL.: 'Targeting T cells against brain tumors with a bispecific ligand-antibody conjugate' INTERNATIONAL JOURNAL OF CANCER vol. 76, no. 5, 29 May 1998, pages 761 - 766, XP002967549
- DATABASE MEDLINE [Online] US NATIONAL LIBRARY OF MEDICINE (BETHESDA, MD, USA) LEAMON C.P. ET AL.: 'Folate-mediated drug delivery: effect of alternative conjugation chemistry', XP002967550 Retrieved from STN Database accession no. 2000143207 & JOURNAL OF DRUG TARGETING vol. 7, no. 3, 1999, pages 157 - 169
- INSEL ET AL.: 'Potential alternations in immunogenicity by combining or simultaneously administering vaccine components' ANNALS OF THE NEW YORK ACADEMY SCIENCES vol. 754, 31 May 1995, pages 35 - 47, XP002967557
- DATABASE CAPLUS [Online] (COLUMBUS, OHIO, USA) STROMINGER ET AL.: 'Drug-receptor interactions: The example of beta-lactam antibiotics', XP002967558 Retrieved from STN Database accession no. 1979:551180 & ADV. PHARMACOL. THER. PROC. INT. CONGR. PHARMACOL. vol. 10, 1979, pages 209 - 223
- DATABASE MEDLINE [Online] US NATIONAL LIBRARY OF MEDICINE (BETHESDA, MD, USA) EASTY ET AL.: 'Up-regulation of ephrin-A1 during melanoma progression', XP002967559 Retrieved from STN Database accession no. 1999434114 & INTERNATIONAL JOURNAL OF CANCER vol. 84, no. 5, 02 October 1999, pages 494 - 501
- DATABASE MEDLINE [Online] US NATIONAL LIBRARY OF MEDICINE (BETHESDA, MD, USA) WALKER-DANIELS ET AL.: 'Overexpression of the EphA2 tyrosine kinase in prostate cancer', XP002967560 Retrieved from STN Database accession no. 2000013158 & PROSTATE vol. 41, no. 4, 01 December 1999, pages 275 - 280
- DATABASE BIOSIS [Online] BIOLOGICAL ABSTRACTS KREMLEV ET AL.: 'Macrophage-mediated inflammation in experimental autoimmune neuritis: The role of the integrin and chemokine interaction', XP002967561 Retrieved from STN Database accession no. 2001:267809 & FASEB JOURNAL vol. 15, no. 4, 15 March 2001, page A682
- DATABASE MEDLINE [Online] US NATIONAL LIBRARY OF MEDICINE (BETHESDA, MD, USA) HAMAOKA T.: 'Future perspectives on tumor-specific immunotherapy using hapten-reactive T cell activity', XP002967562 Retrieved from STN Database accession no. 89271983 & GAN TO KAGAKY RYOHO vol. 16, April 1989, pages 886 - 896
- PISETSKY D.S.: 'Immune activation by bacterial DNA: A new genetic code' IMMUNITY vol. 5, no. 4, October 1996, pages 303 - 310, XP001002520
- BRIGHTBILL H.D. ET AL: 'Host defence mechanisms triggered by microbial lipoproteins through toll-like receptors' SCIENCE vol. 285, 30 July 1999, pages 732 - 736, XP002317651
- SONG L. ET AL: 'Folate-mediated targeting of antisense oligodeoxynucleotides to ovarian cancer cells' PHARMACEUTICAL RESEARCH vol. 15, no. 10, 1998, pages 1540 - 1545
- CITRO G. ET AL: 'Inhibition of leukaemia cell proliferation by folic acid-polylysine-mediated introduction of c-myb antisense oligodeoxynucleotides into HL-60 cells' BRITISH JOURNAL OF CANCER vol. 69, 1994, pages 463 - 467, XP001095764

## Description

### FIELD OF THE INVENTION

This invention relates to a pharmaceutical composition for use in treating disease states characterized by the existence of pathogenic cell populations. More particularly, cell-targeted ligand-immunogen complexes are administered to a diseased host, optionally in combination with an immune system stimulant, to enhance and/or redirect host immune responses to the pathogenic cells.

### BACKGROUND AND SUMMARY OF THE INVENTION

The mammalian immune system provides a means for the recognition and elimination of tumor cells, other pathogenic cells, and invading foreign pathogens. While the immune system normally provides a strong line of defense, there are still many instances where cancer cells, other pathogenic cells, or infectious agents evade a host immune response and proliferate or persist with concomitant host pathogenicity. Chemotherapeutic agents and radiation therapies have been developed to eliminate replicating neoplasms. However, most, if not all, of the currently available chemotherapeutic agents and radiation therapy regimens have adverse side effects because they work not only to destroy cancer cells, but they also affect normal host cells, such as cells of the hematopoietic system. Furthermore, chemotherapeutic agents have limited efficacy in instances where host drug resistance is developed.

Foreign pathogens can also proliferate in a host by evading a competent immune response or where the host immune system has been compromised by drug therapies or by other health problems. Although many therapeutic compounds have been developed, many pathogens are or have become resistant to such therapeutics. The capacity of cancer cells and infectious organisms to develop resistance to therapeutic agents, and the adverse side effects of the currently available anticancer drugs, highlight the need for the development of new therapies specific for pathogenic cell populations with reduced host toxicity.

Researchers have developed therapeutic protocols for destroying cancer cells by targeting cytotoxic compounds specifically to such cells. These protocols utilize toxins conjugated to ligands that bind to receptors unique to or overexpressed by cancer cells in an attempt to minimize delivery of the toxin to normal cells. Using this approach certain immunotoxins have been developed consisting of antibodies directed to specific receptors on pathogenic cells, the antibodies being linked to toxins such as ricin, Pseudomonas exotoxin, Diptheria toxin, and tumor necrosis factor. These immunotoxins target tumor cells bearing the specific receptors recognized by the antibody (Olsnes, S., Immunol. Today, 10, pp. 291-295, 1989; Melby, E.L., Cancer Res., 53(8), pp. 1755-1760, 1993; Better, M.D., PCT Publication No. WO 91/07418, published May 30, 1991).

Another approach for selectively targeting populations of cancer cells or foreign pathogens in a host is to enhance host immune response against the pathogenic cells, thereby avoiding the need for administration of compounds that may also exhibit independent host toxicity. One reported strategy for immunotherapy is to bind antibodies, for example, genetically engineered multimeric antibodies, to the tumor cell surface to display the constant region of the antibodies on the cell surface and thereby induce tumor cell killing by various immune-system mediated processes. (De Vita, V.T., Biologic Therapy of Cancer, 2d ed. Philadelphia, Lippincott, 1995; Soulillou, J.P., U.S. Patent No. 5,672,486). However, this approach has been complicated by the difficulties in defining tumor-specific antigens. Another approach to relying on host immune competency is the targeting of an anti-T cell receptor antibody or anti-Fc receptor antibody to tumor cell surfaces to promote direct binding of immune cells to tumors (Kranz, D.M., U.S. Patent No. 5,547,668). A vaccine-based approach has also been described which relies on a vaccine comprising antigens fused to cytokines, with the cytokine modifying the immunogenicity of the vaccine antigen, and, thus, stimulating the immune response to the pathogenic agent (Pillai, S., PCT Publication No. WO 91/11146, published Feb. 7, 1991). That method relies on indirect modulation of the immune response reported. Another approach for killing unwanted cell populations utilizes IL-2 or Fab fragments of anti-thymocyte globulin linked to antigens to eliminate unwanted T cells; however, based on reported experimental data, the method appears to eliminate only 50% of the targeted cell population, and results in nonspecific cell killing in vivo (i.e., 50% of peripheral blood lymphocytes that are not T cells are also killed (Pouletty, P., PCT Publication No. WO 97/37690, published October 16, 1997)). Thus, there remains a significant need for therapies directed to treatment of disease states characterized by the existence of pathogenic cell populations in an affected host.

Immunity can be categorized as innate or adaptive immunity with adaptive immunity being mediated by T and B cells which exhibit specificity and memory. The innate immune response is an immediate response that can be mediated by phagocytic cells, natural killer cells, T cells, and other cells, and the complement system. Phagocytic cells such as monocytes/macrophages and dendritic cells (antigen-presenting cells) serve dual functions that include killing and degrading of infectious agents or foreign antigens and presenting of components of the ingested pathogens to naive T cells in the context of MHC or MHC-like self antigens. Naive T cells recognize the foreign antigen in the context of MHC or MHC-like antigens and differentiate into mature T_{H}1 or T_{H}2 cells which can then induce adaptive immunity.

Immune cells that mediate innate immunity are capable of distinguishing between self and pathogenic agents by utilizing receptors on the surface of these cells that recognize conserved motifs on pathogens that are not present in higher eukaryotes. One such class of receptors is the toll-like family of receptors which are conserved between insects and humans and were first identified in *Drosophila*.

The toll-like receptor family contains at least ten members (TLRs 1-10) and toll-like receptors are present on the surface of monocytes/macrophages and dendritic cells. The toll-like receptors are activated through recognition of conserved motifs on pathogens, but it is not known whether the activation occurs through direct binding of the conserved motif to the toll-like receptor or through binding to an accessory receptor that interacts with the toll-like receptor. Activation of toll-like receptors results in cytokine secretion and induction of an innate immune response in which naive T cells differentiate into T_{H}1 or T_{H}2 cells. Thus, toll-like receptors may provide a critical link between innate immune recognition and subsequent activation of adaptive immunity.

Molecules capable of activating toll-like receptors may be components of pathogenic agents, such as muramyl dipeptide, LPS, lipopeptides, lipoproteins, peptidoglycan, lipoteichoic acid, lipoarabinomannan, and Zymosan (a yeast cell wall preparation), or may be other molecules endogenous to a host such as fibronectin or heat shock proteins. These molecules activate toll-like receptors through a direct binding event which promotes cytokine secretion from monocytes/macrophages and dendritic cells resulting in an innate immune response, or the toll-like receptor may be activated indirectly through an accessory receptor.

The present invention is directed to the use of composition in a method of eliminating pathogenic cell populations in a host by increasing host immune system recognition of and response to such cell populations. Effectively, the antigenicity of the cellular pathogens is increased to enhance the endogenous immune response-mediated elimination of the population of pathogenic cells. The method avoids or minimizes the use of cytotoxic or antimicrobial therapeutic agents. The method comprises administration of a ligand-immunogen conjugate wherein the ligand is capable of specific binding to a population of pathogenic cells *in vivo* that uniquely expresses, preferentially expresses, or overexpresses a ligand binding moiety, and the immunogen is a molecule capable of activating a toll-like receptor and is capable of eliciting an innate immune response in the host animal. The immune system mediated elimination of the pathogenic cells is directed by the binding of the ligand component of the ligand-immunogen conjugate to a receptor, a transporter, or other surface-presented protein uniquely expressed, overexpressed, or preferentially expressed by the pathogenic cell. A surface-presented protein uniquely expressed, overexpressed, or preferentially expressed by the pathogenic cell is a receptor not present or present at lower amounts on non-pathogenic cells providing a means for selective elimination of the pathogenic cells. At least one additional therapeutic factor, for example, an immune system stimulant, can be co-administered to the host animal to enhance therapeutic efficacy.

In one embodiment, the present method includes the steps of administering ligands capable of high affinity specific binding *in vivo* to cell surface proteins uniquely expressed, preferentially expressed, or overexpressed on the targeted pathogenic cell population, the ligands being conjugated to immunogens capable of activating a toll-like receptor and eliciting an innate immunity in the host animal, and co-administering at least one therapeutic factor that is an endogenous immune response activator. In one preferred embodiment the method involves administering a ligand-immunogen conjugate composition to the host animal wherein the ligand is folic acid or another folate receptor binding ligand (e.g., folic acid analogues or a folate receptor binding ligand unrelated to folate may be used). The ligand is conjugated, for example, by covalent binding, to the immunogen. At least one additional therapeutic factor, not capable of specific binding to the ligand-immunogen complex, but capable of stimulating or enhancing an endogenous immune response, can be administered to the host animal in conjunction with administration of the ligand-immunogen conjugates.

In accordance with another embodiment there is provided a composition for use in a method of enhancing an endogenous immune response-mediated specific elimination of a population of pathogenic cells in a host animal harboring the population wherein the members of the cell population have an accessible binding site for a ligand. The method comprises the step of administering to the host a ligand-immunogen conjugate composition comprising a complex of the ligand and an immunogen wherein the immunogen is a nucleotide and is capable of activating a toll-like receptor and wherein the ligand is folic acid or another folate receptor-binding ligand and administering to the host at least one additional composition comprising a therapeutic factor, wherein the therapeutic factor is a compound capable of stimulating an endogenous immune response wherein the compound does not bind to the ligand-immunogen conjugate.

In one preferred embodiment, there is provided a composition for use in a method of enhancing an endogenous immune response-mediated specific elimination of a population of pathogenic cells in a host animal harboring the population wherein the population preferentially expresses, uniquely expresses, or overexpresses a folate receptor. The method comprises the step of administering to the host a composition comprising a ligand covalently linked to an immunogen, wherein the nucleotide immunogen is capable of activating a toll-like receptor and wherein the ligand is folic acid or another folate receptor-binding ligand.

In still one other embodiment, the targeted pathogenic cell population is a cancer cell population. In another embodiment the targeted cell population is virus-infected cells. In another embodiment the targeted cell population is a population of exogenous organisms such as bacteria, mycoplasma yeast or fungi. In another embodiment the targeted cell population is a population of activated macrophages that mediates a disease state. The ligand-immunogen conjugate binds to the surface of the tumor cells or pathogenic organisms and "labels" the cell members of the targeted cell population with the immunogen, thereby eliciting an immune-mediated response directed at the labeled cell population. The immunogen can be directly recognized by immune cells and direct killing of the pathogenic cells can occur.

Elimination of the foreign pathogens, infected or neoplastic endogenous cells or pathogenic cells can be enhanced by administering a therapeutic factor capable of stimulating an endogenous immune response. In one embodiment the immune stimulant is an interleukin such as IL-2, IL-12, IL-15, IL-18, or an IFN such as IFN-α, IFN-β, or IFN-γ, or GM-CSF. In another embodiment the immune stimulant may be a cytokine composition comprising combinations of cytokines, such as IL-2, IL-12 or IL-15 in combination with IFN-α, IFN-β, or IFN-γ, or GM-CSF, or any effective combination thereof, or any other effective combination of cytokines. The above-identified cytokines stimulate T_{H}1 responses, but cytokines that stimulate T_{H}2 responses may also be used, such as IL-4, IL-10, IL-11, or any effective combination thereof. Also, combinations of cytokines that stimulate T_{H}1 responses along with cytokines that stimulate T_{H}2 responses may be used.

In another embodiment, a pharmaceutical composition is provided. The pharmaceutical composition comprises therapeutically effective amounts of a ligand-immunogen conjugate wherein the immunogen is capable of activating a toll-like receptor and wherein the ligand is folic acid or another folate receptor-binding ligand, and a pharmaceutically acceptable carrier therefor.

In still one other embodiment, there is provided a pharmaceutical composition comprising therapeutically effective amounts of a ligand-immunogen conjugate wherein the immunogen is capable of activating a toll-like receptor and wherein the ligand is folic acid or another folate receptor-binding ligand, a therapeutic factor wherein the therapeutic factor is a compound capable of stimulating an endogenous immune response wherein the compound does not bind to the ligand-immunogen conjugate, and a pharmaceutically acceptable carrier therefor. In one embodiment the pharmaceutical composition is in a parenteral prolonged release dosage form. In another embodiment the therapeutic factor is an immune stimulant comprising a compound selected from the group consisting of any interleukin including IL-2, IL-4, IL-10, IL-11, IL-12, IL-15, and IL-18, an IFN such as IFN-α IFN-β, or IFN-γ, and GM-CSF, or combinations thereof.

### BRIEF DESCRIPTION OF THE DRAWINGS

Fig. 1 Muramyl dipeptide and muramyl dipeptide-folate therapy for tumor-implanted mice.
Fig. 2 CpG and CpG-folate therapy for tumor-implanted mice.

### DETAILED DESCRIPTION OF THE INVENTION

Compositions are provided for the therapeutic treatment of a host with a disease state mediated by pathogenic cells or a host infected with pathogenic organisms. The methods result in enhancement of the immune response-mediated elimination of pathogenic cell populations by rendering/labeling the pathogenic cells antigenic resulting in their recognition and elimination by the host immune system. The method employs a ligand-immunogen conjugate capable of high affinity binding to cancer cells or other pathogenic agents or pathogenic cells. The high affinity binding can be inherent to the ligand and it may be modified (enhanced) by the use of a chemically modified ligand or from the particular chemical linkage between the ligand and the immunogen that is present in the conjugate. The method can also utilize combination therapy by employing the ligand-immunogen conjugate and an additional therapeutic factor capable of enhancing immune response-mediated elimination of the pathogenic cell populations.

The composition of the present invention is utilized to enhance an endogenous immune response-mediated elimination of a population of pathogenic cells in a host animal harboring the population of pathogenic cells. The invention is applicable to populations of pathogenic cells that cause a variety of pathologies such as cancer, inflammation, autoimmune diseases, and infectious diseases. Thus, the population of pathogenic cells can be a cancer cell population that is tumorigenic, including benign tumors and malignant tumors, or it can be non-tumorigenic. The cancer cell population can arise spontaneously or by such processes as mutations present in the germline of the host animal or somatic mutations, or it may be chemically-, virally-, or radiation-induced. The invention can be utilized to treat such cancers as carcinomas, sarcomas, lymphomas, Hodgekin's disease, melanomas, mesotheliomas, Burkitt's lymphoma, nasopharyngeal carcinomas, leukemias, and myelomas. The cancer cell population can include oral, thyroid, endocrine, skin, gastric, esophageal, laryngeal, pancreatic, colon, bladder, bone, ovarian, cervical, uterine, breast, testicular, prostate, rectal, kidney, liver, and lung cancers.

The population of pathogenic cells can also be an exogenous pathogen or a cell population harboring an exogenous pathogen, e.g., a virus. The present invention is applicable to such exogenous pathogens as bacteria, fungi, viruses, mycoplasma, and parasites. Infectious agents that can be treated with the present invention are any art-recognized infectious organisms that cause pathogenesis in an animal, including such organisms as bacteria that are gram-negative or gram-positive cocci or bacilli, DNA and RNA viruses, including DNA viruses such as papilloma viruses, parvoviruses, adenoviruses, herpesviruses and vaccinia viruses, and RNA viruses, such as arenaviruses, coronaviruses, rhinoviruses, respiratory syncytial viruses, influenza viruses, picomaviruses, paramyxoviruses, reoviruses, retroviruses, and rhabdoviruses. Of particular interest are bacteria that are resistant to antibiotics such as antibiotic-resistant *Streptococcus* species and *Staphlococcus* species, or bacteria that are susceptible to antibiotics, but cause recurrent infections treated with antibiotics so that resistant organisms eventually develop. Such organisms can be treated with the ligand-immunogen conjugates of the present invention in combination with lower doses of antibiotics than would normally be administered to a patient to avoid the development of these antibiotic-resistant bacterial strains. The present invention is also applicable to any fungi, mycoplasma species, parasites, or other infectious organisms that cause disease in animals. Examples of fungi that can be treated with the method of the present invention include fungi that grow as molds or are yeast-like, including, for example, fungi that cause diseases such as ringworm, histoplasmosis, blastomycosis, aspergillosis, cryptococcosis, sporotrichosis, coccidioidomycosis, paracoccidio-idomycosis, and candidiasis. The present invention can be utilized to treat parasitic infections including infections caused by somatic tapeworms, blood flukes, tissue roundworms, ameba, and *Plasmodium, Trypanosoma, Leishmania*, and *Toxoplasma* species. Parasites of particular interest are those that express folate receptors and bind folate; however, the literature is replete with reference to ligands exhibiting high affinity for infectious organisms. For example, penicillins and cephalosporins known for their antibiotic activity and specific binding to bacterial cell wall precursors can similarly be used as ligands for preparing ligand-immunogen conjugates for use in accordance with this invention. The ligand-immunogen conjugates of the invention can also be directed to a cell population harboring endogenous pathogens wherein pathogen-specific antigens are preferentially expressed on the surface of cells harboring the pathogens, and act as receptors for the ligand with the ligand specifically binding to the antigen.

Additionally, the population of pathogenic cells can be a population of activated macrophages that mediates a disease state. Activated macrophages often overexpress cell surface proteins such as the folate receptor. The activated macrophages may aggravate or cause such disease states as ulcerative colitis, Crohn's disease, rheumatoid arthritis, osteomyelitis, artherosclerosis, graft versus host disease, psoriasis, osteoporosis, sarcoidosis, multiple sclerosis, and other inflammatory and autoimmune diseases. The activated macrophage cell population can also be a population of activated macrophages infected with such pathogens as *Salmonella, Shigella*, and *Tuberculosis* species. The activated macrophages are targeted for elimination as a result of binding of the ligand-immunogen conjugates to the activated macrophage cell surface.

The composition of the present invention can be used for both human clinical medicine and veterinary applications. Thus, the host animals harboring the population of pathogenic organisms and treated with ligand-immunogen conjugates can be humans or, in the case of veterinary applications, can be laboratory, agricultural, domestic, or wild animals. The present invention can be applied to host animals including humans, laboratory animals such rodents (e.g., mice, rats, hamsters, etc.), rabbits, monkeys, chimpanzees, domestic animals such as dogs, cats, and rabbits, agricultural animals such as cows, horses, pigs, sheep, goats, and wild animals in captivity such as bears, pandas, lions, tigers, leopards, elephants, zebras, giraffes, gorillas, dolphins, and whales.

The ligand-immunogen conjugate is preferably administered to the host animal parenterally, e.g., intradermally, subcutaneously, intramuscularly, intraperitoneally, or intravenously. Alternatively, the conjugate can be administered to the host animal by other medically useful processes, such as oral administration, and any effective dose and suitable therapeutic dosage form, including prolonged release dosage forms, can be used. The method of the present invention can be used in combination with surgical removal of a tumor, radiation therapy, chemotherapy, or biological therapies such as other immunotherapies including monoclonal antibody therapy, treatment with immunomodulatory agents, adoptive transfer of immune effector cells, treatment with hematopoietic growth factors, cytokines and vaccination.

In accordance with the present invention, the ligand component of the ligand-immunogen conjugates is folic acid, and other folate receptor-binding molecules including folate receptor binding ligands unrelated to folate.

The binding site for the ligand is the folate receptor. The invention further contemplates the use of combinations of ligand-immunogen conjugates to maximize targeting of the pathogenic cells for elimination by an innate immune response.

Acceptable immunogens for use in the present invention are nucleotide immunogens capable of activating a member of the toll-like family of receptors (e.g., TLR2, TLR4, TLR5, TLR6, TLR9, or TLR10) and eliciting an innate immune response. Suitable immunogens for use in the invention include ligands capable of activating a toll-like receptor such as nucleotides, such as CpG nucleotides.

The ligands and immunogens of the invention can be conjugated by utilizing any art-recognized method of forming a complex. This can include covalent, ionic, or hydrogen bonding of the ligand to the immunogen, either directly or indirectly via a linking group such as a divalent linker. The conjugate is typically formed by covalent bonding of the ligand to the immunogen through the formation of amide, ester or imino bonds between acid, aldehyde, hydroxy, amino, or hydrazo groups on the respective components of the complex. In a preferred embodiment, the ligand is folic acid, an analog of folic acid, or any other folate-receptor binding molecule, and the folate ligand is conjugated to the immunogen by a procedure that utilizes trifluoroacetic anhydride to prepare γ-esters of folic acid via a pteroyl azide intermediate. This preferred procedure results in the synthesis of a folate ligand, conjugated to the immunogen only through the γ-carboxy group of the glutamic acid groups of folate wherein the γ-conjugate binds to the folate receptor with high affinity, avoiding the formation of mixtures of an α-conjugate and the γ-conjugate. Alternatively, pure α-conjugates can be prepared from intermediates wherein the γ-carboxy group is selectively blocked, the α-conjugate is formed and the γ-carboxy group is subsequently deblocked using art-recognized organic synthesis protocols and procedures. Alternatively, folate receptor blinding ligands unrelated to folate can be used.

The ligand-immunogen conjugates of the invention enhance an endogenous immune response-mediated elimination of a population of pathogenic cells. The endogenous immune response includes a cell-mediated immune response, and any other immune response endogenous to the host animal. For example, the endogenous immune response can involve toll-like receptor expressing immune cells such as macrophages and dendritic cells that play significant roles in the innate immune response by serving as antigen-presenting cells. The delivered ligand-immunogen conjugates function as cross-linking agents for recruitment of toll-like receptor expressing immune cells to pathogenic cells. The direct recognition of the delivered immunogen by toll-like receptors expressed on the surface of antigen-presenting cells can result in the production of cytokines such as IL-12 and IL-18, which induce naive T cells to differentiate into T_{H}1 cells. Alternatively, the immunogen can activate a toll-like receptor indirectly through interaction with an accessory protein, leading ultimately to an innate immune response involving differentiation of naive T cells into T_{H}1 cells. Therefore, activation of toll-like receptors provides an innate mechanism by which the ligand-immunogen conjugates preferentially activate cell-mediated immunity against the population of pathogenic cells.

The immunogen may also be internalized by antigen-presenting cells through interaction with toll-like receptors or by phagocytosis or endocytosis resulting in the processing of the immunogen and presentation to naive T cells in the context of MHC or MHC-like antigens (e.g., CD1) on the surface of antigen-presenting cells. As a result of antigen presentation, antigen-presenting cells induce naive T cells to differentiate into T_{H}1 cells, but a T_{H}2 response can also be induced. The polarization of the immune response toward a T_{H}1 or T_{H}2 response depends on the cytokines released during the interaction of T cells with antigen-presenting cells. The T_{H}1 and T_{H}2 responses result from antigen presentation in combination with release of stimulatory cytokines from antigen-presenting cells (e.g., IL-12 and IL-18 to induce the T_{H}1 response and IL-4 to induce the T_{H}2 response). Effector cytokines can also be released from T_{H}1 cells (e.g., IFN-γ) and T_{H}2 cells (e.g., IL-4, IL-5, IL-10, and IL-13) to further promote T cell differentiation. It is also contemplated that the innate immune response will employ the secretion of cytokines that regulate such processes as the multiplication and migration of immune cells.

Toll-like receptor ligands can induce an humoral response as a result of induction of innate immunity. In this case, toll-like receptor ligands can bind to toll-like receptors on dendritic cells (a type of antigen-presenting cell) promoting maturation of the dendritic cells. Mature dendritic cells then migrate to draining lymph nodes where they induce adaptive immunity (e.g., an humoral response) by stimulating T lymphocytes to secrete cytokines that promote humoral responses.

If adaptive immunity (i.e., an humoral response) is induced consequent to induction of innate immunity, it is contemplated that antibodies would be directed to the tumor cells or infectious organisms by binding to ligand-immunogen conjugates which preferentially bind to these invading cells or organisms and that the pathogenic cells would be killed by complement-mediated lysis, ADCC, antibody-dependent phagocytosis, or antibody clustering of receptors. The cytotoxic process can also involve other types of immune responses, such as cell-mediated immunity, as well as secondary responses that arise when the attracted antigen-presenting cells phagocytose the unwanted cells and present natural tumor antigens or antigens of foreign pathogens to the immune system for elimination of the cells or organisms bearing the antigens.

At least one additional composition comprising a therapeutic factor can be administered to the host in combination or as an adjuvant to the above-detailed methodology, to enhance the endogenous immune response-mediated elimination of the population of pathogenic cells, or more than one additional therapeutic factor can be administered. The therapeutic factor is a compound capable of stimulating an endogenous immune response or other therapeutic factor capable of complementing the efficacy of the administered ligand-immunogen complex. The method of the invention can be performed by administering to the host, in addition to the above-described conjugates, compounds or compositions capable of stimulating an endogenous immune response including cytokines or immune cell growth factors such as interleukins 1-18, stem cell factor, basic FGF, EGF, G-CSF, GM-CSF, FLK-2 ligand, HILDA, MIP-1α, TGF α TGF β, M-CSF, IFN α, IFN β, IFN γ, soluble CD23, LIF, and combinations thereof.

Therapeutically effective combinations of these cytokines may also be used. In a preferred embodiment, for example, therapeutically effective amounts of IL-2, for example, in amounts ranging from 5000 IU/dose /day to 500,000 IU/dose/day in a multiple dose daily regimen, and IFN-α, for example, in amounts ranging from 7500 IU/dose/day to 150,000 IU/dose/day in a multiple dose daily regimen, are used along with ligand-immunogen conjugates to eliminate pathogenic cells in a host animal harboring such a population of cells. In another preferred embodiment IL-12 and IFN-α are used in therapeutically effective amounts along with the ligand-immunogen conjugates, and in yet another preferred embodiment IL-15 and IFN-α are used in therapeutically effective amounts along with the ligand-immunogen conjugates. In an alternate preferred embodiment IL-2, IFN-α or IFN-γ, and GM-CSF are used in combination along with the ligand-immunogen conjugates. Preferably, the therapeutic factor(s) used, such as IL-2, IL-12, IL-15, IL-18, IFN-α IFN-γ, and GM-CSF, including combinations thereof, activate(s) natural killer cells and/or T cells (i.e., T_{H}1 cells). Alternatively, the therapeutic factor or combinations thereof, including an interleukin in combination with an interferon and GM-CSF, can activate other immune effector cells such as macrophages, B cells, neutrophils, LAK cells or the like, or the therapeutic factor can activate T_{H}2 cells (e.g., IL-4, IL-10, or IL-11). The invention also contemplates the use of any other effective combination of cytokines including combinations of other interleukins and interferons and colony stimulating factors.

Chemotherapeutic agents, which are cytotoxic themselves and can work to enhance tumor permeability, can be used in combination with the ligand-immunogen conjugates and cytokines in the method of the invention and such chemotherapeutic agents include adrenocorticoids, alkylating agents, antiandrogens, antiestrogens, androgens, estrogens, antimetabolites such as cytosine arabinoside, purine analogs, pyrimidine analogs, and methotrexate, busulfan, carboplatin, chlorambucil, cisplatin and other platinum compounds, tamoxiphen, taxol, cyclophosphamide, plant alkaloids, prednisone, hydroxyurea, teniposide, antibiotics such as mitomycin C and bleomycin, nitrogen mustards, nitrosureas, vincristine, vinblastine, inflammatory and proinflammatory agents, and any other art-recognized chemotherapeutic agent. Other therapeutic agents that can be administered adjuvant to the administration of the present conjugates, include penicillins, cephalosporins, vancomycin, erythromycin, clindamycin, rifampin, chloramphenicol, aminoglycosides, gentamicin, amphotericin B, acyclovir, trifluridine, ganciclovir, zidovudine, amantadine, ribavirin, and any other art-recognized antimicrobial compound.

The elimination of the population of pathogenic cells will comprise a reduction or elimination of tumor mass or of pathogenic organisms or pathogenic cells resulting in a therapeutic response. In the case of a tumor, the elimination can be an elimination of cells of the primary tumor or of cells that have metastasized or are in the process of dissociating from the primary tumor. A prophylactic treatment to prevent return of a tumor after its removal by any therapeutic approach including surgical removal of the tumor, radiation therapy, chemotherapy, or biological therapy is also contemplated in accordance with this invention. The prophylactic treatment can be an initial treatment with the ligand-immunogen conjugate, such as treatment in a multiple dose daily regimen, and/or can be an additional treatment or series of treatments after an interval of days or months following the initial treatments(s).

The invention is also directed to pharmaceutical compositions comprising an amount of a ligand-immunogen conjugate effective to "label" a population of pathogenic cells in a host animal for specific elimination by an endogenous immune response. Optionally, the composition further comprises an amount of a compound capable of stimulating an endogenous immune response wherein the compound does not bind to the ligand-immunogen conjugate. The compound is effective to enhance the elimination of the pathogenic cells. The pharmaceutical composition contains therapeutically effective amounts of the ligand-immunogen conjugate and the therapeutic factor and the factor may comprise a cytokine such as IL-2, IL-4, IL-10, IL-11, IL-12, IL-15, or IL-18 or combinations of cytokines, including IL-2, IL-4, IL-10, IL-11, IL-12, IL-15, or IL-18 and interferons such as IFN-α or IFN-γ and combinations of interferons, interleukins, and colony stimulating factors, such as GM-CSF.

The unitary daily dosage of the ligand-immunogen conjugate can vary significantly depending on the host condition, the disease state being treated, the molecular weight of the conjugate, its route of administration and tissue distribution, and the possibility of co-usage of other therapeutic treatments such as radiation therapy. The effective amount to be administered to a patient is based on body surface area, patient weight, and physician assessment of patient condition. An effective dose can range from 1 ng/kg to 1 mg/kg, more preferably from 1 µg/kg to 500 µg/kg, and most preferably from 1 µg/kg to 100 µg/kg.

Any effective regimen for administering the ligand-immunogen conjugate and the therapeutic factor, if included, can be used. For example, the ligand-immunogen conjugate and therapeutic factor can be administered as single doses, or they can be divided and administered as a multiple-dose daily regimen. Further, a staggered regimen, for example, one to three days per week can be used as an alternative to daily treatment, and for the purpose of defining this invention such intermittent or staggered daily regimen is considered to be equivalent to every day treatment and within the scope of this invention. In a preferred embodiment of the invention the host is treated with multiple injections of the ligand-immunogen conjugate and the therapeutic factor to eliminate the population of pathogenic cells. In one embodiment, the host is injected multiple times (preferably 2 up to 50 times) with the ligand-immunogen conjugate, for example, at 12-72 hour intervals or at 48-72 hour intervals. Additional injections of the ligand-immunogen conjugate can be administered to the patient at an interval of days or months after the initial injections(s) and the additional injections prevent recurrence of disease. Alternatively, the initial injection(s) of the ligand-immunogen conjugate may prevent recurrence of disease.

The therapeutic factor can be administered to the host animal prior to, after, or at the same time as the ligand-immunogen conjugate and the therapeutic factor may be administered as part of the same composition containing the conjugate or as part of a different composition than the ligand-immunogen conjugate. Any such therapeutic composition containing the therapeutic factor at a therapeutically effective dose can be used in the present invention. Additionally, more than one type of ligand-immunogen conjugate can be used. For example, the host animal can be treated with muramyl dipeptide and taxol or a CpG nucleotide linked to the same or different ligands in a co-dosing protocol. In the case of chemotherapeutic and antimicrobial agents, the therapeutic factor can be administered at a suboptimal dose along with the ligand-immunogen conjugate in a combination therapy to avoid development of resistance to the chemotherapeutic or antimicrobial agent by the host animal.

The ligand-immunogen conjugate and the therapeutic factor are preferably injected parenterally and such injections can be intraperitoneally injections, subcutaneous injections, intramuscular injections, intravenous injections or intrathecal injections. The ligand-immunogen conjugate and the therapeutic factor can also be delivered using a slow pump. Examples of parenteral dosage forms include aqueous solutions of the active agent, in an isotonic saline, 5% glucose or other well-known pharmaceutically acceptable liquid carries such as liquid alcohols, glycols, esters, and asides. The parenteral dosage form in accordance with this invention can be in the forum of a reconstitutable lyophilazate comprising the dose of ligand-immunogen conjugate and therapeutic factor. In one preferred aspect of the present embodiment, any of a number of prolonged release dosage forms known in the art can be administered such as, for example, the biodegradable carbohydrate matrices described in U.S. Patents Nos. 4,713,249; 5,266,333; and 5,417,982.

Oral ingestion can also be used to administer the ligand-immunogen conjugates and therapeutic factor and such dosage forms include syrups, sprays, or other liquid dosage forms, a gol-seal, or a capsule or caplet. Buccal and sublingual administration comprises contacting the oral and pharyngeal mucosa of the patient with the dose of ligand-immunogen conjugate and therapeutic factor either in a pharmaceutically acceptable liquid dosage form, such as a syrup or a spray, or in a saliva-soluble dosage form which is held in the patient's mouth to form a saliva solution in contact with the oral and pharyngeal mucosa. Exemplary of saliva-soluble dosage forms are lozenges, tablets.

The ligand-immunogen conjugates and therapeutic factor intended for buccal or sublingual administration in accordance with the present invention are administered to the patient in a dosage form adapted to promote contact with the patient's oral and pharyngeal mucosa. Thus, the dosage form can be in the form of a liquid solution such as a syrup, spray, or other liquid dosage form to be administered and used by the patient in a manner which promotes contact of the with oral mucosal tissues. Alternatively, the conjugates with therapeutic factor can be administered by oral ingestion wherein the compounds are formulated into a syrup to be swallowed by the patient and not hold in the mouth. Syrups for either use may be flavored or unflavored and may be formulated using a buffered aqueous solution as a base with added caloric or non-caloric sweeteners, flavor oils and pharmaceutically acceptable surfactant/dispersants. Other liquid dosage forms, including solutions or sprays can be prepared in a similar manner and can be administered buccally, sublingually, or by oral ingestion.

Preferably, the conjugates with therapeutic factor for buccal/sublingual administration in the present invention are formulated into a solid dosage form, such as a lozenge or a tablet. This formulation preferably contains a saliva-soluble carrier and can optionally contain desirable excipients, such as buffers, or tableting aids.

Lozenges for use in accordance with this invention can be prepared, for example, by art-recognized techniques for forming compressed tablets where the conjugates and therapeutic factor are dispersed on a compressible solid carrier, optionally combined with any appropriate tableting aids such as a lubricant (e.g., magnesium-stearate) and is compressed into tablets. The solid carrier component for such tableting formulations can be a saliva-soluble solid, such as a cold water-soluble starch or a monosaccharide or disaccharide, so that the lozenge will readily dissolve in the mouth. The pH of the above-described formulations can range from 4 to 8.5. Lozenges for use in accordance with the present invention can also be prepared utilizing other art-recognized solid unitary dosage formulation techniques.

Tablets for use in accordance with this invention can be prepared in a manner similar to that described for preparation of lozenges or by other art-recognized techniques for forming compressed tablets such as chewable vitamins. Suitable solid carrier components for tableting include mannitol, microcrystalline cellulose, carboxymethyl cellulose, and dibasic calcium phosphate.

Solid dosage forms for oral ingestion administration include such dosage forms as caplets, capsules, and gel-seals. Such solid dosage forms can be prepared using standard tableting protocols and excipients to provide conjugate and therapeutic factor-containing capsules, caplets, or gel-seals. Any of the solid dosage forms for use in accordance with the invention, including lozenges and tablets, can be in a form adapted for sustained release.

### REFERENCE EXAMPLE 1

### EFFECT OF FOLATE-MURAMYL DIPEPTIDE CONJUGATES ON SURVIVAL OF MICE WITH LUNG TUMOR IMPLANTS

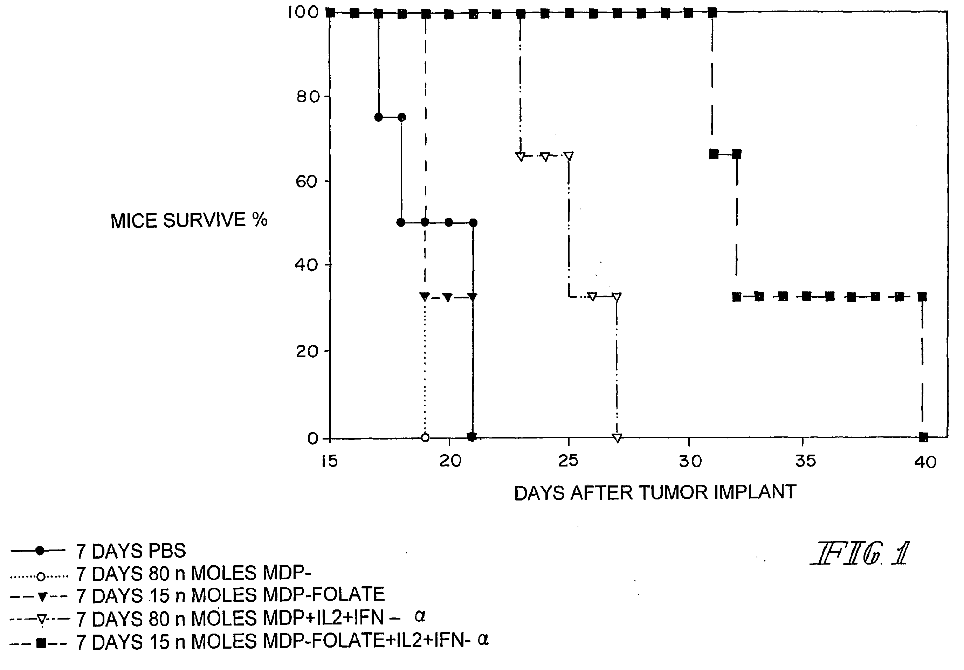

Female Balb/c mice were injected on day 0 with 5 x 10⁵ M109 cells, a syngeneic lung cancer cell line that expresses high levels of the folate receptor. In an effort to reduce the time required to obtain long-term survival data, the tumor cells were implanted intraperitoneally close to the liver. Cancer loci were then allowed to attach and grow. The animals were then injected with PBS (control) or were coinjected with folate-muramyl dipeptide (MDP) (15 nmoles/kg), IL-2 (5,000 IU/dose), and IFN-α (25,000 U/dose) on days 7, 8, 9, 11, and 14 after tumor cell implantation. Folate was conjugated to MDP via a gamma carboxyl-linked ethylene diamine bridge. Additional animals were injected with 80 nmoles/kg MDP, 15 nmoles/kg folate-MDP, or 80 nmoles/kg MDP and IL-2 and IFN-α at the concentrations specified above. The efficacy of this immunotherapy was evaluated by monitoring survival as a function of time of folate-MDP treated mice compared to control animals. As shown in Fig. 1, control mice all died by day 21 whereas mice treated with MDP-folate + IL-2 + IFN-α survived to day 40. Additionally, the capacity of folate-MDP in combination with IL-2 and IFN-α to promote long-term survival of tumor-bearing mice is strongly synergistic as the combination of IL-2 and IFN-α had little effect on long-term survival of mice and folate-MDP alone had a negligible effect on the survival of the mice.

### EXAMPLE 2

### EFFECT OF FOLATE-CpG CONJUGATES ON SURVIVAL OF MICE WITH LUNG TUMOR IMPLANTS

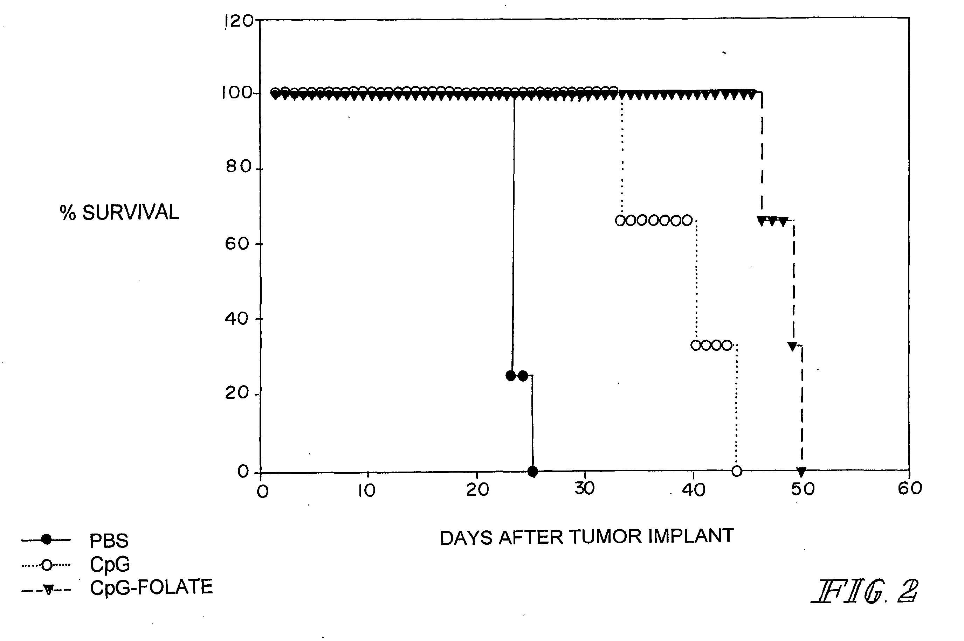

Female Balb/c mice were injected on day 0 with 5 x 10⁵ M109 cells, a syngeneic lung cancer cell line that expresses high levels of the folate receptor. In an effort to reduce the time required to obtain long-term survival data, the tumor cells were implanted intraperitoneally close to the liver. Cancer loci were then allowed to attach and grow. The animals were then injected with PBS (control), CpG (1500 nmoles/kg) or were injected with folate-CpG (1500 nmoles/kg) on days 7 and 8 after tumor cell implantation. Folate was conjugated to 5'-amino CpG via a peptide bridge. The efficacy of this immunotherapy was evaluated by monitoring survival as a function of time. As shown in Fig. 2, control mice all died by day 25 whereas mice treated with CpG survived to day 44 and mice treated with folate-CpG survived to day 50.

## Claims

1. A pharmaceutical composition in a parenteral dosage form comprising therapeutically effective amounts of a ligand-immunogen conjugate wherein the immunogen activates a toll-like receptor, wherein the immunogen is a nucleotide and wherein the ligand is folic acid or another folate receptor-binding ligand and a pharmaceutically acceptable carrier therefore, **characterized** that the composition further comprises IL-2, IL-4, IL-10, IL-11, IL-12, IL-15, IL-18, or combinations thereof.

2. The pharmaceutical composition of claim 1 further comprising IL-2, IL-4, IL-10, IL-11, IL-12, IL-15, IL-18, or combinations thereof, in combination with IFN-α or IFN-γ.

3. A pharmaceutical composition in a parenteral dosage form comprising therapeutically effective amounts of a ligand-immunogen conjugate wherein the immunogen activates a toll-like receptor, wherein the immunogen is a nucleotide and wherein the ligand is folic acid or another folate receptor-binding ligand and a pharmaceutically acceptable carrier therefore, **characterized** that the composition further comprises IL-2, IL-4, IL-10, IL-11, IL-12, IL-15, IL-18, IFN-α, IFN-γ, GM-CSF, or combinations thereof.

4. The pharmaceutical composition of claims 1 to 3 wherein the ligand is conjugated to the immunogen through bonding comprising covalent, ionic, or hydrogen bonding.

5. The pharmaceutical composition of any preceding claim, wherein the immunogen is a CpG nucleotide.

6. The pharmaceutical composition of any preceding claim in a parenteral prolonged release dosage form.

7. Use of a pharmaceutical composition in a parenteral dosage form comprising therapeutically effective amounts of a ligand-immunogen conjugate wherein the immunogen activates a toll-like receptor and wherein the immunogen is a nucleotide and wherein the ligand is folic acid or another folate receptor-binding ligand in the preparation of a medicament for treating cancer, **characterized** that the medicament further comprises IL-2, IL-4, IL-10, IL-11, IL-12, IL-15, IL-18, or combinations thereof.

8. The use of claim 6, wherein the medicament further comprises IL-2, IL-4, IL-10, IL-11, IL-12, IL-15, IL-18, or combinations thereof, in combination with IFN-α or IFN-γ.

9. Use of a pharmaceutical composition in a parenteral dosage form comprising therapeutically effective amounts of a ligand-immunogen conjugate wherein the immunogen activates a toll-like receptor and wherein the immunogen is a nucleotide and wherein the ligand is folic acid or another folate receptor-binding ligand in the preparation of a medicament for treating cancer, **characterized** that the medicament further comprises IL-2, IL-4, IL-10, IL-11, IL-12, IL-15, IL-18, IFN-α, IFN-γ, GM-CSF, or combinations thereof.

10. The use of claims 7 to 9, wherein the ligand is conjugated to the immunogen through bonding comprising covalent, ionic, or hydrogen bonding.

11. The use of claims 7 to 10, wherein the immunogen is a CpG nucleotide.

12. The use of any of preceding claims 7 to 11 wherein the medicament is in a parenteral prolonged release dosage form.

13. A pharmaceutical composition of claims 1-6 in a parenteral dosage form comprising therapeutically effective amounts of a ligand-immunogen conjugate for use in activating a toll-like receptor wherein the immunogen is a nucleotide and wherein the ligand is folic acid or another folate receptor-binding ligand, and a pharmaceutically acceptable carrier therefor in combination with IL-2, IL-4, IL-10, IL-11, IL-12, IL-15, IL-18, IFN-α, IFN-γ, GM-CSF, or combinations thereof.

## Patentansprüche

1. Pharmazeutische Zusammensetzung in Form einer parenteralen Dosis, welche therapeutisch wirksame Mengen eines Liganden-Immunogen-Konjugats, wobei das Immunogen einen Toll-like Rezeptor aktiviert, wobei das Immunogen ein Nukleotid ist und wobei der Ligand Folsäure oder ein anderer Folatrezeptoren bindender Ligand ist, und einen pharmazeutisch verträglichen Träger dafür umfasst, **dadurch gekennzeichnet, dass** die Zusammensetzung ferner IL-2, IL-4, IL-10, IL-11, IL-12, IL-15, IL-18 oder Kombinationen derselben umfasst.

2. Pharmazeutische Zusammensetzung nach Anspruch 1, ferner umfassend IL-2, IL-4, IL-10, IL-11, IL-12, IL-15, IL-18 oder Kombinationen derselben in Kombination mit IFN-α oder IFN-γ.

3. Pharmazeutische Zusammensetzung in Form einer parenteralen Dosis, welche therapeutisch wirksame Mengen eines Liganden-Immunogen-Konjugats, wobei das Immunogen einen Toll-like Rezeptor aktiviert, wobei das Immunogen ein Nukleotid ist und wobei der Ligand Folsäure oder ein anderer Folatrezeptoren bindender Ligand ist, und einen pharmazeutisch verträglichen Träger dafür umfasst, **dadurch gekennzeichnet, dass** die Zusammensetzung ferner IL-2, IL-4, IL-10, IL-11, IL-12, IL-15, IL-18, IFN-α, IFN-γ, GM-CSF oder Kombinationen derselben umfasst.

4. Pharmazeutische Zusammensetzung nach Anspruch 1 bis 3, wobei der Ligand durch eine Bindung, die eine kovalente Bindung, eine Ionenbindung oder eine Wasserstoffbindung umfasst, mit dem Immunogen konjugiert ist.

5. Pharmazeutische Zusammensetzung nach einem der vorangegangenen Ansprüche, wobei das Immunogen ein CpG-Nukleotid ist.

6. Pharmazeutische Zusammensetzung nach einem der vorangegangenen Ansprüche in Form einer parenteralen Dosis mit anhaltender Freisetzung.

7. Verwendung einer pharmazeutischen Zusammensetzung in Form einer parenteralen Dosis, welche therapeutisch wirksame Mengen eines Liganden-Immunogen-Konjugats, wobei das Immunogen einen Toll-like Rezeptor aktiviert und wobei das Immunogen ein Nukleotid ist und wobei der Ligand Folsäure oder ein anderer Folatrezeptoren bindender Ligand ist, umfasst, zur Herstellung eines Medikaments zur Behandlung von Krebs, **dadurch gekennzeichnet, dass** das Medikament ferner IL-2, IL-4, IL-10, IL-11, IL-12, IL-15, IL-18 oder Kombinationen derselben umfasst.

8. Verwendung nach Anspruch 7, wobei das Medikament ferner IL-2, IL-4, IL-10, IL-11, IL-12, IL-15, IL-18 oder Kombinationen derselben in Kombination mit IFN-α oder IFN-γ umfasst.

9. Verwendung einer pharmazeutischen Zusammensetzung in Form einer parenteralen Dosis, welche therapeutisch wirksame Mengen eines Liganden-Immunogen-Konjugats, wobei das Immunogen einen Toll-like Rezeptor aktiviert und wobei das Immunogen ein Nukleotid ist und wobei der Ligand Folsäure oder ein anderer Folatrezeptoren bindender Ligand ist, umfasst, zur Herstellung eines Medikaments zur Behandlung von Krebs, **dadurch gekennzeichnet, dass** das Medikament ferner IL-2, IL-4, IL-10, IL-11, IL-12, IL-15, IL-18, IFN-α, IFN-γ, GM-CSF oder Kombinationen derselben umfasst.

10. Verwendung nach einem der Ansprüche 7 bis 9, wobei der Ligand durch eine Bindung, die eine kovalente Bindung, eine Ionenbindung oder eine Wasserstoffbindung umfasst, mit dem Immunogen konjugiert ist.

11. Verwendung nach einem der Ansprüche 7 bis 10, wobei das Immunogen ein CpG-Nukleotid ist.

12. Verwendung nach einem der Ansprüche 7 bis 11, wobei das Medikament in Form einer parenteralen Dosis mit anhaltender Freisetzung vorliegt.

13. Pharmazeutische Zusammensetzung nach einem der Ansprüche 1 bis 6 in Form einer parenteralen Dosis, welche therapeutisch wirksame Mengen eines Liganden-Immunogen-Konjugats zur Verwendung zum Aktivieren eines Toll-like Rezeptors, wobei das Immunogen ein Nukleotid ist und wobei der Ligand Folsäure oder ein anderer Folatrezeptoren bindender Ligand ist, und einen pharmazeutisch verträglichen Träger dafür in Kombination mit IL-2, IL-4, IL-10, IL-11, IL-12, IL-15, IL-18, IFN-α, IFN-γ, GM-CSF oder Kombinationen derselben umfasst.

## Revendications

1. Composition pharmaceutique dans une forme pharmaceutique parentérale comprenant des quantités thérapeutiquement efficaces d'un conjugué ligand-immunogène dans lequel l'immunogène active un récepteur de type Toll, dans lequel l'immunogène est un nucléotide et dans lequel le ligand est l'acide folique ou un autre ligand de liaison de récepteur d'acide folique et par conséquent un support pharmaceutiquement acceptable, **caractérisée en ce que** la composition comprend en outre IL-2, IL-4, IL-10, IL-11, IL-12, IL-15, IL-18, ou des combinaisons de celles-ci.

2. Composition pharmaceutique selon la revendication 1, comprenant en outre IL-2, IL-4, IL-10, IL-11, IL-12, IL-15, IL-18, ou des combinaisons de celles-ci, en combinaison avec IFN-α ou IFN-γ.

3. Composition pharmaceutique dans une forme pharmaceutique parentérale comprenant des quantités thérapeutiquement efficaces d'un conjugué ligand-immunogène dans lequel l'immunogène active un récepteur de type Toll, dans lequel l'immunogène est un nucléotide et dans lequel le ligand est l'acide folique ou un autre ligand de liaison de récepteur d'acide folique et par conséquent un support pharmaceutiquement acceptable, **caractérisée en ce que** la composition comprend en outre IL-2, IL-4, IL-10, IL-11, IL-12, IL-15, IL-18, IFN-α, IFN-γ, GM-CSF, ou des combinaisons de ceux-ci.

4. Composition pharmaceutique selon les revendications 1 à 3, dans laquelle le ligand est conjugué avec l'immunogène par des liaisons comprenant des liaisons covalentes, ioniques ou hydrogène.

5. Composition pharmaceutique selon l'une quelconque des revendications précédentes, dans laquelle l'immunogène est un nucléotide CpG.

6. Composition pharmaceutique selon l'une quelconque des revendications précédentes dans une forme pharmaceutique à libération prolongée parentérale.

7. Utilisation d'une composition pharmaceutique dans une forme pharmaceutique parentérale comprenant des quantités thérapeutiquement efficaces d'un conjugué ligand-immunogène dans lequel l'immunogène active un récepteur de type Toll et dans lequel l'immunogène est un nucléotide et dans lequel le ligand est l'acide folique ou un autre ligand de liaison de récepteur d'acide folique dans la préparation d'un médicament destiné à traiter le cancer, **caractérisée en ce que** le médicament comprend en outre IL-2, IL-4, IL-10, IL-11, IL-12, IL-15, IL-18, ou des combinaisons de celles-ci.

8. Utilisation selon la revendication 6, dans laquelle le médicament comprend en outre IL-2, IL-4, IL-10, IL-11, IL-12, IL-15, IL-18, ou des combinaisons de celles-ci, en combinaison avec IFN-α ou IFN-γ.

9. Utilisation d'une composition pharmaceutique dans une forme pharmaceutique parentérale comprenant des quantités thérapeutiquement efficaces d'un conjugué ligand-immunogène dans lequel l'immunogène active un récepteur de type Toll et dans lequel l'immunogène est un nucléotide et dans lequel le ligand est l'acide folique ou un autre ligand de liaison de récepteur d'acide folique dans la préparation d'un médicament destiné à traiter le cancer, **caractérisée en ce que** le médicament comprend en outre IL-2, IL-4, IL-10, IL-11, IL-12, IL-15, IL-18, IFN-α, IFN-γ, GM-CSF, ou des combinaisons de ceux-ci.

10. Utilisation selon les revendications 7 à 9, dans laquelle le ligand est conjugué avec l'immunogène par des liaisons comprenant des liaisons covalentes, ioniques ou hydrogène.

11. Utilisation selon les revendications 7 à 10, dans laquelle l'immunogène est un nucléotide CpG.

12. Utilisation selon l'une quelconque des revendications précédentes 7 à 11, dans laquelle le médicament est dans une forme pharmaceutique à libération prolongée parentérale.

13. Composition pharmaceutique selon les revendications 1 à 6 dans une forme pharmaceutique parentérale comprenant des quantités thérapeutiquement efficaces d'un conjugué ligand-immunogène pour une utilisation pour activer un récepteur de type Toll dans lequel l'immunogène est un nucléotide et dans lequel le ligand est l'acide folique ou un autre ligand de liaison de récepteur d'acide folique, et par conséquent un support pharmaceutiquement acceptable, en combinaison avec IL-2, IL-4, IL-10, IL-11, IL-12, IL-15, IL-18, IFN-α, IFN-γ, GM-CSF, ou des combinaisons de ceux-ci.
